# EUROPEAN PATENT SPECIFICATION

(11) **EP 1 479 407 B1**
(45) Date of publication and mention of the grant of the patent: **01.03.2006**
(21) Application number: 04019652.9
(22) Date of filing: 22.12.1999
(51) Int. Cl.: A61M 25/01

(54) **Sensor and guide wire assembly**
Sensor und Führungsdrahteinheit
Capteur et ensemble de fil de guidage

(30) Priority: 23.12.1998 US 113810 P; 09.07.1999 US 349980
(43) Date of publication of application: 24.11.2004
(62) Divisional of application: 99965684.6
(73) Proprietor: RADI MEDICAL SYSTEMS AB, 754 50 Uppsala (SE)
(72) Inventor: Hammarström, Ola, 540 17 Lerdala (SE); von Malmborg, Per, 755 97 Uppsala (SE); Tenerz, Lars, 756 46 Uppsala (SE); Benkowski, Per, 755 98 Uppsala (SE)
(74) Representative: Lindgren, Anders

(56) References cited:
- WO-A-90/01294
- US-A- 5 226 423
- US-A- 5 715 827

## Description

The present invention relates to a sensor and guide wire assembly for intravascular pressure measurements having improved handling properties in the vessels in which it is to be inserted.

A sensor mounted on a guide wire suitable for use in intravascular pressure measurements is disclosed in International Patent Application No. WO 90/01294 and in US Patents No. Re. 35,648, and No. 5,715,827 (closest prior art). One known sensor and guide wire assembly comprises a core wire having segments of varying thicknesses in at least the distal region of the core wire. In situations where it is desirable to attach a sensor having electrical leads connected thereto to the core wire, the core wire is enclosed in a tube, and the electrical leads run parallel inside the tube along the core wire. The core wire may, but need not, extend all the way to the proximal end of the tube. If the sensor is a pressure sensor, it is preferable to provide some sort of stiffening member for the sensor in order to avoid mechanical artifacts due to, for example, bending. It is known to provide a short, protective tube segment enclosing the sensor to stiffen and protect the sensor region of the guide wire. A stiffening member of this type has several limitations, however.

A first drawback of a protective tube segment, of the type indicated above, is that the formation of a joint between the coils and protective tube segment is difficult to make, and mismatch often occurs between coil and tube. Put another way, it is difficult to obtain good concentric matching.

A second drawback of a protective tube segment relates to the manufacturing process, which becomes more complicated because an additional structure, i.e., the protective tube segment, must be assembled as part of the sensor and guide wire assembly. A conventional means of attaching the protective tube segment to the sensor is with glue or another suitable adhesive, but this attachment method renders the joint non-optimal from a mechanical point of view. Further, the thermal expansion coefficient of the adhesive may differ from that of the materials used in the remaining components of the sensor and guide wire assembly, which can cause problems. The protective tube segment also makes the sensor region relatively stiff, and the length of the protective tube segment cannot be made as short as desirable.

A third drawback of a protective tube segment is that the tube segment constitutes an asymmetric and non-flexible or stiff portion that hinders bending at turns in a vessel.

A fourth drawback of a protective tube segment is that the inner diameter of the tube segment puts limitations on how the segmenting of the wire can be achieved.

The difficulties suggested in the proceeding are not intended to be exhaustive but rather are among many which tend to reduce the effectiveness and manufacturing efficiencies of conventional sensor and guide wire assemblies. Other noteworthy problems may also exist; however, those presented above should be sufficient to demonstrate that such assemblies appearing in the past will admit to worthwhile improvement.

### SUMMARY OF THE INVENTION

Thus, there exists a need for a sensor and guide wire assembly having improved bending characteristics in the region of the sensor.

An object of the invention is therefore to provide a sensor and guide wire assembly that exhibits a smoother transition where the sensor element is placed and that is easier to produce from a manufacturing point of view.

A preferred embodiment of the invention, which is intended to accomplish the foregoing objects, includes a sensor and guide wire assembly having a mounting member. The mounting member preferably comprises an enlarged portion having a sensor receptacle or recess in which the sensor is mounted. This assembly yields an overall stiffer and mechanically robust device, without the drawbacks associated with a separate, protective tube segment as outlined in the above discussion of conventional devices. The assembly of the invention does not require a protective tube segment because the enlarged portion, having a sensor receptacle or recess formed therein, provides a protected mounting site for the sensor.

A sensor and guide wire assembly in accordance with the invention may comprise a core wire having a distal end, a proximal end, and a plurality of sections of different cross sections and different flexibilities. At least one of the sections has an enlarged portion, and the enlarged portion has a sensor receptacle therein. A tube encloses the core wire over at least a fraction of its length such that the core wire, including the enlarged portion, extends out from a distal end of the tube. The tube is configured to enable the sensor and guide wire assembly to be inserted into an artery and to be passed to a measurement site inside a patient's body. A sensor is mounted in the sensor receptacle of the enlarged portion of the core wire. A first coil is arranged to enclose a first portion of the core wire extending out from the distal end of the tube. The first coil is located nearer to the proximal end of the core wire than the sensor. A second coil is arranged to enclose a second portion of the core wire extending out from the distal end of the tube. The second coil is located nearer to the distal end of the core wire than the sensor. The first coil and the second coil each preferably have an outer diameter that is essentially the same as an outer diameter of the enlarged portion.

Additional objects and advantages of the invention will be set forth in the description that follows, and in part will be obvious from the description, or may be learned by practice of the invention. The objects and advantages of the invention may be realized and obtained by means of the instrumentalities and combinations particularly pointed out in the appended claims.

### BRIEF DESCRIPTION OF THE DRAWINGS

The accompanying drawings, which are incorporated in and constitute a part of the specification, illustrate a presently preferred embodiment of the invention, and, together with the general description given above and the detailed description of the preferred embodiment given below, serve to explain the principles of the invention.
Figs. 1 and 1a illustrate a longitudinal side view, in cross section, of a full length of a sensor and guide wire assembly according to the invention;
Fig. 1b is a cross-sectional view taken along line b-b in Fig. 1a;
Fig. 2 is a detail side view, in cross section, showing an alternative way of mounting the sensor;
Fig. 3 is a detail side view, in cross section, showing a way of threadedly attaching the coils onto an enlarged portion of a core wire of the sensor and guide wire assembly;
Fig. 4a is a detail side view, in cross section, showing another way of attaching the coils to the core wire;
Fig 4b is a detail side view, in cross section, showing a variation of the embodiment of Fig. 4a, where one coil functions to protect the sensor;
Fig. 5a is a detail side view, in cross section, of an embodiment where a mounting slot for a sensor extends through the enlarged portion;
Fig. 5b is a cross-sectional view taken along line b-b in Fig 5a;
Fig. 6 is a detail side view, in cross section, showing the tip , or distal end, of the sensor and guide wire assembly;
Fig. 7 is a detail side view, in cross section, illustrating one way of attaching a coil to the core wire and to the tube of the sensor and guide wire assembly;
Fig. 8 is a detail side view, in cross section, illustrating an alternative way of attaching a coil to the core wire and to the tube;
Fig. 9 is a detail side view, in cross section, illustrating a further way of attaching a coil to the core wire and to the tube;
Fig. 10 is a detail side view, in cross section, illustrating a still further way of attaching a coil to the core wire and to the tube;
Fig. 11 is a detail side view, in cross section, illustrating another way of attaching a coil to the core wire and to the tube;
Fig. 12 is a detail side view, in cross section, illustrating a further alternative way of attaching a coil to the core wire and to the tube; and
Fig. 13 is a detail side view, in cross section, illustrating still another way of attaching a coil to the core wire and to the tube.

### DETAILED DESCRIPTION OF THE PREFERRED EMBODIMENT

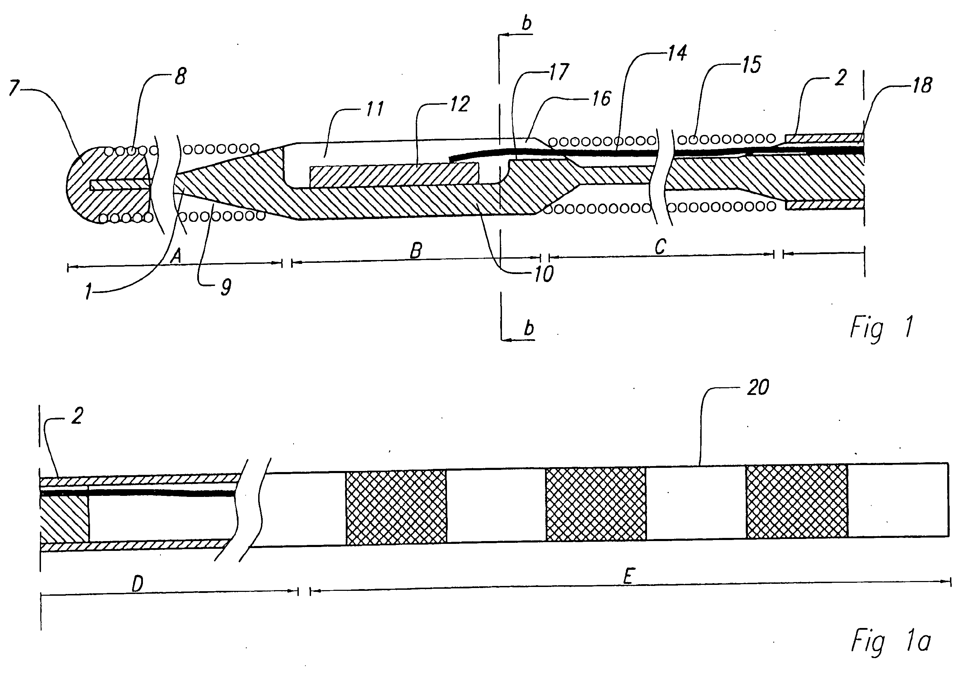

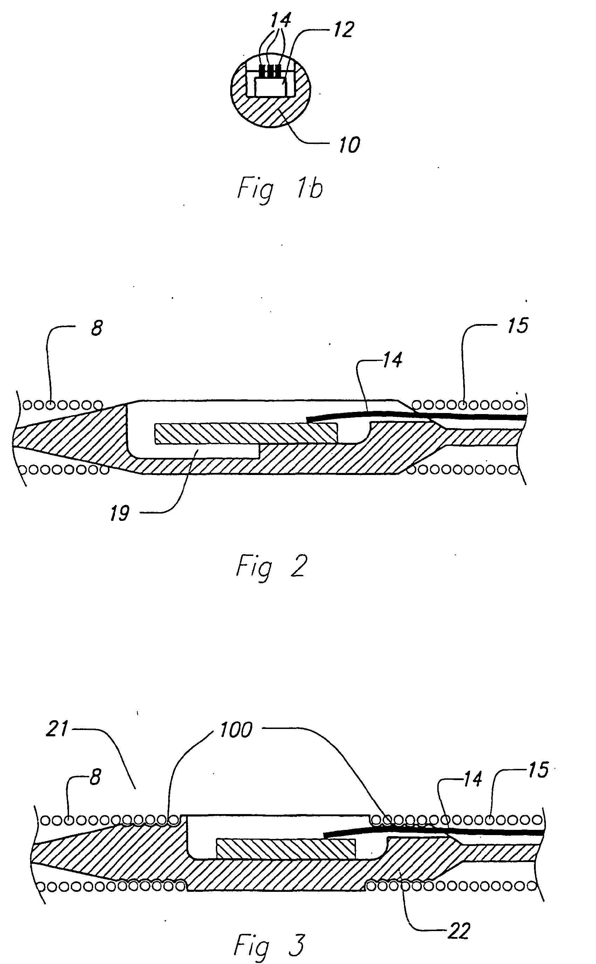

Referring now to the drawings, wherein like numerals indicate like parts, and initially to Figs. 1 and 1a, there will be seen a preferred embodiment of the sensor and guide wire assembly. As shown in Figs. 1 and 1a, the assembly generally comprises a core wire 1, a tube 2 in which the core wire 1 at least partially is inserted, at least one coil 8, 15, partially covering a distal portion of the core wire, and a sensor element 12 attached to the core wire 1 in a suitable mounting arrangement (to be described below). The core wire 1 has an enlarged portion 10. The cross section of the enlarged portion 10 has a major dimension that is larger than the major dimension of the cross sections of the remaining portions of the core wire 1. In the case of a cylindrical core wire, the major dimension of the enlarged portion 10 is represented by the diameter of the circular cross section of the core wire, and that diameter is larger than the diameters of the remaining portions of the core wire. In the embodiment of Figs. 1 and 1a, the distal end of the core wire 1, anchored in tip 7 of the assembly, has a smaller diameter than the diameter of the enlarged portion 10.

For illustrative purposes, the assembly has been divided into five sections, A-E, in Figs. 1 and 1a. Section A is the most distal portion of the assembly, i.e., that portion which is going to be inserted farthest into the vessel, and section E is the most proximal portion. Section E is provided with a male proximal connector 20. In a preferred embodiment, section A is about 10-50 mm; section B is about 0.5-5 mm; section C is about 200-400 mm; section D is about 500-3500 mm; and section E is about 5-50 mm in length. The diameter of the guide wire varies between 0.25-2 mm. For use in coronary arteries, the diameter is normally 0.35 mm.

Section A preferably includes a radiopaque coil 8, which is made of, for example, platinum or a platinum alloy, usable as a position marker during manipulation of the guide wire. At the very distal end of section A, there is provided a tip 7 having a hemispherical outer shape. The tip 7 may be attached to the coil 8 by welding, soldering, or other suitable attachment methods.

The core wire 1 has an enlarged portion 10 in section B. In the transition area between sections A and B, the proximal end of the coil 8 is attached to the enlarged portion 10 of the core wire 1 with glue, solder, or another suitable adhesive. Alternatively, the coil 8 may be threaded onto the enlarged portion 10 (further details of the coil attachment will be given below). Another coil 15 is attached to a proximal end of the enlarged portion 10. The coil 15 extends over section C for providing flexibility in this region of the core wire 1 and protection of the cables 14.

In the enlarged portion 10, there is provided a sensor receptacle, preferably a slot 11, in which a sensor 12 is mounted. The sensor may, for example, be a pressure sensor. The enlarged portion 10 of the core wire 1, in which the sensor 12 is mounted, decreases the stress exerted on the sensor 12 during sharp vessel turns. Moreover, the enlarged portion 10, by virtue of the provision of the slot 11, protects the sensor 12 from being mechanically destroyed during handling of the core wire 1 or by contact with the vessel wall. The side walls inside the slot 11 should extend a certain distance above the upper surface of the sensor 12 in order to provide the protection desired.

The enlarged portion 10 is preferably made by removing material from a metal wire having the nominal diameter of the enlarged portion so as to form smaller diameter segments extending distally and proximally of the enlarged portion. The machining of the wire can be made by various methods, centerless grinding being a preferred method, although other methods are possible.

Alternatively, the enlarged portion may be made by attaching a short tube segment on a core wire, for example, by soldering. A depression forming a mounting receptacle for the sensor may be made by spark machining. In this way, the overall structure will closely resemble the embodiment of Figs. 1 and 1a.

Attached to the sensor 12 are signal transmitting cables 14, the number of which may vary, depending on the design of the sensor. In the embodiment shown, there are three cables (see Fig. 1b). These cables 14 are, for example, attached to the sensor 12 on bond pads (not shown) by bonding or by another suitable technique. The points of attachment of the cables 14 to the sensor 12 are preferably protected from the environment, i.e., from blood or other body fluids. Normally, this may be achieved by filling the slot 11 with silicon rubber or other polymer material to provide adequate protection from such fluids and other environmental impact.

The slot 11 preferably has a width and depth dimension of approximately 50-250 µm and a length dimension of approximately is 50-2500 µm.

As can be seen in Fig. 1a, the slot 11 extends in the proximal direction of the core wire 1 to transform into a shallower recess 16 in which the cables 14 rest. The slot 11 and recess 16 are configured so that the coil 15 does not interfere with or damage the delicate cables 14 when the coil 15 is attached. The relatively shallow recess 16 is defined by a shelf 17 machined in the core wire 1. The main reason for this shelf 17 is that, if the slot 11 were to extend further in the proximal direction of the core wire 1 than the distal edge of the relatively shallow recess 16, the strength of the enlarged portion 10 of the core wire 1 would be inadequate at the transition between sections B and C.

A recess 18, similar to recess 16, is formed in the core wire 1 at the transition between sections C and D, where the core wire 1 is inserted in the tube 2. This recess 18 is provided for the protection of the cables 14, so that they will not be damaged, for example, during assembly.

In the shown embodiment, the core wire 1 extends into the tube 2 only over a relatively short distance in section D. It may be glued in place in the tube 2, although other methods of attachment are possible. However, it is also conceivable, and indeed may be preferable, to let the core wire 1 extend over the entire length of the assembly, all the way up to the proximal connector 20. In this case, it may be necessary to join two pieces to form the core wire, since it may be unfavorable to form a long core wire with enlarged portions via the grinding method. Simply too much metal working would be necessary.

The distal end of the enlarged portion 10, located in section A, preferably is tapered to form a slightly conical extension and functions as an attachment surface for the coil 8. This extension is narrowed down to form a thin wire, which is anchored in the tip 7 of the assembly. The successive tapering of the core wire 1 in section A towards the tip results in a front portion of the guide wire assembly that becomes progressively more flexible nearing the tip 7. This tapering may be obtained by grinding of the metal core wire. It should be noted that the attachment surface of the core wire need not be conical, but rather may be formed as a flattened portion to which the coil and tip may attach.

The coils 8, 15 may be attached to the various parts of the assembly by different methods as will be described.

In Fig. 2, an embodiment of the invention is shown wherein a further depression or recess 19 has been formed in the bottom of the enlarged portion 10. The purpose of this recess 19 is to ensure that the distal, pressure sensitive part of the sensor will not experience any mechanical stress, which otherwise could be induced by stress in the enlarged portion 10 if the core wire 1 were bent in the region of sections A-B-C, especially at the transitions between these sections.

As can be clearly seen in Figs. 1 and 2, the enlarged portion 10 has an outer diameter that is essentially equal to the outer diameter of the coils 8, 15. This will render the diameter of the entire sensor and guide wire assembly the same over the length thereof. The coils and the enlarged portion also will be centered on the longitudinal axis. Furthermore, the outer surface will exhibit no "edges" at the joints between the coils and the enlarged portion that potentially could cause problems during insertion by becoming stuck or hooked in irregularities.

As indicated above, there are several alternative methods of attaching the coils 8, 15 to the enlarged portion 10 and to the tube 2, respectively. Also, there are various alternatives for attachment of the core wire 1 to the tube 2. Some of these alternatives will now be described.

In Fig. 3, an embodiment is shown wherein the coils 8, 15 are threaded (at 100) onto the enlarged portion 10. To this end, the enlarged portion 10 has been made to have distal and proximal extensions 21, 22, respectively, each having a reduced diameter relative to the major part of the enlarged portion 10. The threads 100 are most conveniently made by EDM or micromachining. The number of threads is not critical, but 4-10 threads would be suitable.

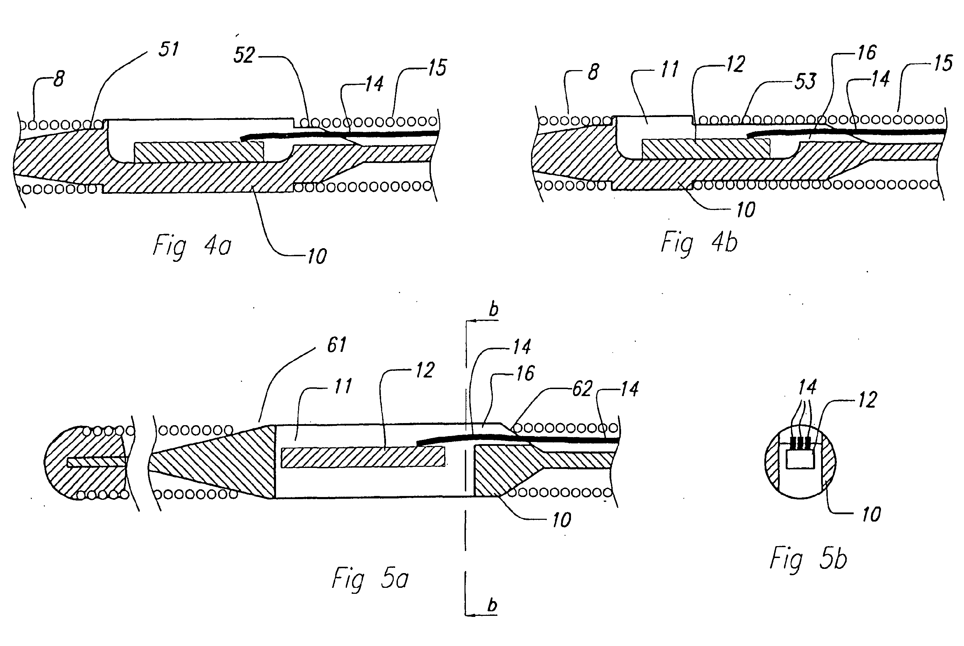

In Fig. 4a, there are provided distal and proximal extensions 51, 52, respectively, each having a reduced diameter relative to the major part of the enlarged portion 10. The coils 8, 15 are attached thereto, for example, by welding, soldering, or gluing. However, these extensions 51, 52 may be shorter than the extensions 21, 22 of the embodiment in Fig. 3. In both the embodiment of Fig. 3 and of Fig. 4a, it is important that the outer diameter of the coil sections are substantially the same as that of the enlarged portion 10.

In Fig. 4b, the enlarged portion 10 has been made with a smaller diameter over a larger fraction of its length, to form a proximal extension 53, such that the proximal coil 15, when attached thereto, covers a large fraction (as much as up to 2/3 or more) of the slot 11. Thereby, further protection for the sensor 12 is provided.

Also in these embodiments, the slot 11 extends in the proximal direction so as to form a recess 16, in which the cables 14 may rest for protective purposes.

In Figs. 5a and 5b, an embodiment is shown wherein the slot 11 extends all the way through the enlarged portion 10. In order to fix the sensor 12 in position inside the slot 11, the slot 11 is filled with a material such as silicon rubber or other material. The silicon has a protective effect. Also, in this embodiment, there is provided a recess 16 for accommodating the cables 14.

A particular feature of the enlarged portion 10 of Figs. 5a and 5b is its tapered extensions 61 and 62, respectively, in distal and proximal directions. As indicated in the introduction, the provision of a protective tube for the sensor tends to cause manufacturing problems because mismatches between the coil and the tube may occur. The quality of the joints also varies considerably, yielding variations in the flexibility in the sensor region from one assembly to another. By forming the enlarged portion 10 with tapered surfaces 61, 62, it becomes relatively easy to mount the coils 8, 15, since the coils 8, 15 may be guided onto the enlarged portion 10 to their mounting positions.

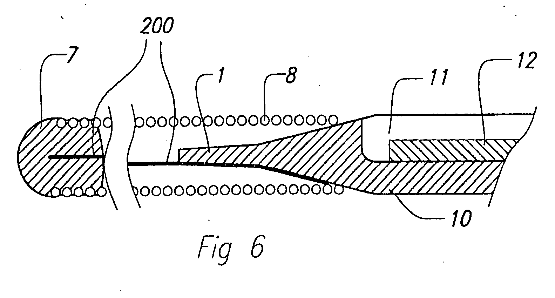

In Fig. 6, it is shown how the core wire 1 can be secured in the tip 7 by attaching a separate wire 200 to the core wire 1 and anchoring it in the tip 7.

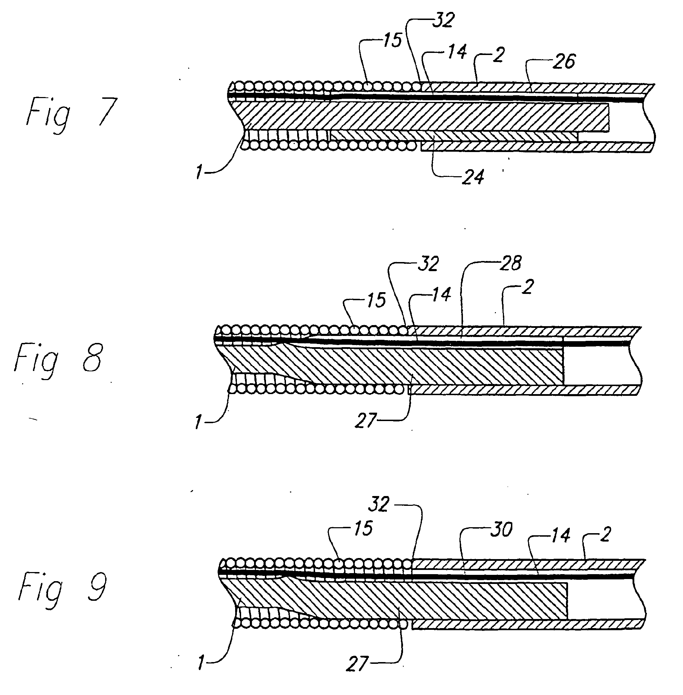

Now a number of possibilities of attaching the core wire 1 and coil 15 to the tube 2 will be described with reference to Figs. 7-13. In all these figures, the core wire 1 is shown to extend only a short distance into the tube 2. However, it may be desirable, from a point of view of providing enough bending strength, to let the core wire 1 extend even all the way through the tube 2 to its proximal end, where a connector (20 as shown in Fig. 1) is provided.

Fig. 7 illustrates an embodiment wherein the core wire 1 has been enclosed in, or inserted in, a tube segment 24 that has an outer diameter corresponding to or being slightly smaller than the inner diameter of the tube 2, in order to obtain a tight fit between the core wire 1 and the tube 2. A longitudinal recess 26 is made in the tube segment 24 for the accommodation of the cables 14. The cross section of the tube segment 24 may resemble a C-shape, where the cables 14 run in the opening of the "C." The material of the tube segment 24 is preferably, but not limited to, polyimide. The material thickness of the tube 2 is preferably substantially the same as the diameter of the wire making up the coil 15. Thereby, the outer diameter of the coil 15 will be essentially equal to the outer diameter of the tube 2. The part of the tube segment 24 that extends out from an edge 32 of the tube 2 functions as a mount for the coil 15. The coil 15 is pushed onto the tube segment 24 and secured thereto by gluing, welding, soldering, or any other suitable technique.

In Fig. 8, a tight fit between core wire 1 and tube 2 is obtained by increasing the diameter of the core wire 1 to form a core wire segment 27, the diameter of which corresponds to or is slightly smaller than the inner diameter of the tube 2. For accommodation of the cables 14, a recess 28 is made in the core wire segment 27 by grinding or other machining methods, or some other suitable method. The core wire segment 27 extends out from an edge 32 of the tube 2 to provide a mounting site for the coil 15, similar to the embodiment of Fig. 7.

Fig. 9 shows an embodiment, similar to the embodiment of Fig. 8, the difference being that, instead of providing a recess, core wire material has been removed to obtain a planar surface defining a shelf 30. The shelf 30 provides a space for accommodating said cables 14. Whereas the windings of the coil 15 are not visible along the length of the recess 28 from the coil's point of attachment to the tube 2 to a distal end of the recess 28, indicating that the material of the enlarged portion 27 blocks the coil windings from view, the coil windings are visible in the same region in Fig. 9, which illustrates a planar shelf 30 instead of a recess 28. The coil 15 is attached to the core wire segment 27, as in Fig. 8.

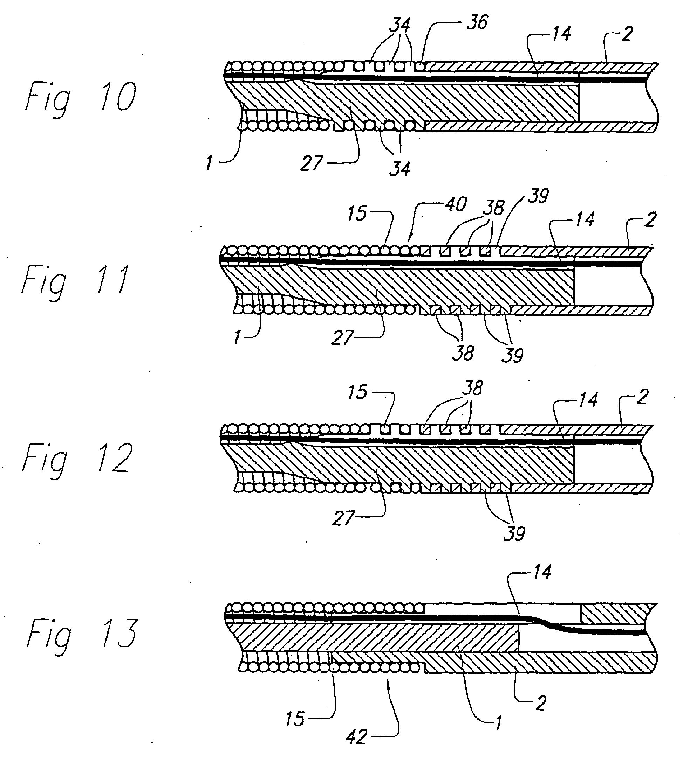

Fig. 10 illustrates an alternative attachment of the coil 15 to what is disclosed in Fig. 8. Here, the core wire segment 27 has been provided with threads 34 in which the coil windings 36 may be accommodated. Threading in this way may of course be supplemented with gluing or soldering or the like.

Fig. 11 illustrates an embodiment wherein the tube 2 has been machined at its distal end such that a spiral like structure 38 is obtained. The core wire segment 27 of the core wire 1 is provided with threads 39, adapted to cooperate with the spiral like structure 38, to form an engagement between the tube 2 and core wire 1. The coil 15 is attached to the non-threaded part of the core wire segment 27, the non-threaded portion forming a mounting site 40 for the coil 15.

Fig. 12 is a further development of the embodiment of Fig. 11, wherein both the coil 15 and the tube 2 are threaded onto threads 39 of the core wire segment 27. Of course, the attachment of the coil 15 and the threads 39 of the core wire segment 27 to the tube 2 can be supplemented with glue or solder.

In the embodiment of Fig. 13, the distal end of the tube 2 has a smaller outer diameter and less thickness than a remaining portion of the tube 2 so that the distal end may provide a mounting site 42 for the coil 15.

## Claims

1. A sensor and guide wire assembly, comprising
a core wire (1) having a distal end, a proximal end, and an enlarged portion (10) at the distal end, said enlarged portion being provided with a sensor mounting member in the form of a slot (11);
a sensor (12) mounted in said sensor mounting member of said enlarged portion (10) of said core wire (1);
**characterized in that**
said mounting member comprises an essentially cylindrical member, said slot (11) being formed therein, and adapted to house said sensor, and **in that**
the side walls inside the slot (11) extend a certain distance above the upper surface of the sensor (12) in order to provide adequate protection of said sensor from mechanical interference.

2. The sensor and guide wire assembly as recited in claim 1, wherein the slot (11) has a width and depth dimension in the range 50-250 µm, and a length dimension in the range 50-2500 µm.

3. The sensor and guide wire assembly as recited in claim 1, wherein said sensor (12) is secured in place by embedding said sensor in a soft material selected from the group consisting of silicon rubber, latex, and other polymer materials.

4. The sensor and guide wire assembly as recited in claim 1, wherein a further depression (19) has been formed in the bottom of the enlarged portion (10), beneath the distal end of the sensor (12), to ensure that the distal, pressure sensitive part of the sensor will not experience any mechanical stress.

5. The sensor and guide wire assembly as recited in claim 1, wherein said slot (11) in which said sensor is mounted extends through said enlarged portion.

6. The sensor and guide wire assembly as recited in claim 1, comprising a tube (2) enclosing said core wire (1) over at least a fraction of a length of said core wire such that said core wire (1) extends out from a distal end of said tube (2), said tube being configured to enable said sensor and guide wire assembly to be inserted into an artery and to be passed to a measurement site inside a body.

7. The sensor and guide wire assembly as recited in claim 6, comprising wiring (14) extending from said sensor (12) through at least a portion of said tube (2) in a direction toward a proximal end of said tube; and
a tube segment (24) having an outer diameter essentially the same as an inner diameter of said tube, said tube segment being positioned between said core wire (1) and said tube (2) in an interference fit with said tube to secure said core wire in place in said tube, and said tube segment being configured to allow passage of said wiring through said portion of said tube.

8. The sensor and guide wire assembly as recited in claim 7, wherein a portion of said tube segment (24) extends out from said distal end of said tube, and said sensor and guide wire assembly further comprises a coil (15) mounted to said portion of said tube segment extending out from said distal end of said tube, said tube segment having an outer diameter essentially equal to an outer diameter of said tube (2).

9. The sensor and guide wire assembly as recited in claim 6, wherein said core wire is mounted to said tube in an interference fit such that an outer diameter of said core wire is essentially equal to an inner diameter of said tube.

10. The sensor and core guide assembly as recited in any preceding claim, wherein said core wire includes a longitudinal recess (28) therein for accommodating said wiring.

11. The sensor and guide wire assembly as recited in any of claims 1-10, wherein said core wire includes a planar surface (30) for accommodating said wiring.

12. The sensor and guide wire assembly as recited in any of claims 1-10, further comprising a coil (8) mounted to a portion of said core wire extending out from a distal end of said tube.

13. The sensor and guide wire assembly as recited in claim 6, wherein said core wire is threadedly engaged to said tube, said core wire including external threads (39) that are complementary to a spiral-like structure formed at said distal end of said tube such that said spiral-like structure of said tube threadedly engages at least some of said threads of said core wire.

14. The sensor and guide wire assembly as recited in claim 14, further comprising a coil having coil windings threadedly engaging distal ones of said threads (39) of said core wire.

## Patentansprüche

1. Sensor und Führungsdrahteinheit umfassend
einen Kerndraht (1), der ein distales Ende, ein proximales Ende und einen aufgeweiteten Bereich (10) an dem distalen Ende aufweist, wobei der aufgeweitete Bereich mit einem Sensorhalterungselement in Form eines Schlitzes (11) versehen ist;
ein Sensor (12), der in dem Sensorhalterungselement des aufgeweiteten Bereichs (10) des Kerndrahtes (1) montiert ist;
**dadurch gekennzeichnet, dass**
das Halterungselement ein im wesentlichen zylinderförmiges Element umfasst, in dem der Schlitz (11) eingeformt ist und das so ausgeprägt ist, dass es den Sensor aufnehmen kann, und dass
Seitenwände innerhalb des Schlitzes (11) sich in einen bestimmten Abstand über der oberen Oberfläche des Sensors (12) erstrecken, um einen angemessenen Schutz des Sensors vor medizinischer Interferenz bereitzustellen.

2. Sensor und Führungsdrahteinheit gemäß Anspruch 1, wobei der Schlitz (11) eine Weiten- und Tiefenabmessung im Bereich von 50-25 µm und eine Längenabmessung im Bereich von 50-2500 µm aufweist.

3. Sensor und Führungsdrahteinheit gemäß Anspruch 1, wobei der Sensor (12) an seinem Ort befestigt ist durch Einbetten des Sensors in ein weiches Material, das aus der Gruppe umfassend Silikongummi, Latex und anderen polymeren Materialien ausgewählt ist.

4. Sensor und Führungsdrahteinheit gemäß Anspruch 1, wobei eine weitere Mulde (19) in den Boden des aufgeweiteten Bereiches (10) unterhalb des distalen Endes des Sensors (12) eingeformt, ist um sicherzustellen, dass der distale, drucksensitive Teil des Sensors nicht irgendeine mechanische Belastung erfährt.

5. Sensor und Führungsdrahteinheit gemäß Anspruch 1, wobei der Schlitz (11), in dem der Sensor montiert ist, sich entlang des aufgeweiteten Bereichs erstreckt.

6. Sensor und Führungsdrahteinheit gemäß Anspruch 1, umfassend eine Röhre (2), die den Kerndraht (1) über zumindest einen Teil der Länge des Kerndrahtes einschließt, so dass sich der Kerndraht (1) von einem distalen Ende der Röhre (2) aus erstreckt, wobei die Röhre so ausgebildet ist, dass sie es ermöglicht, dass der Sensor und die Führungsdrahteinheit in eine Arterie eingebracht werden kann und zu einem Messort innerhalb des Körpers geführt werden kann.

7. Sensor und Führungsdrahteinheit gemäß Anspruch 6, umfassend eine Verdrahtung (14), die sich von dem Sensor (12) aus durch zumindest einen Teil der Röhre (2) in einer Richtung zu dem proximalen Ende der Röhre hin erstreckt; und
ein Röhrensegment (24), das einen äußeren Durchmesser aufweist, der im Wesentlichen der gleiche ist wie ein innerer Durchmesser der Röhre, wobei das Röhrensegment zwischen dem Kerndraht (1) und der Röhre (2) positioniert ist in einer Presspassung mit der Röhre, um den Kerndraht an seinem Ort in der Röhre zu halten und dass das Röhrensegment so ausgestaltet ist, dass die Verdrahtung durch den Bereich der Röhre hindurchgehen kann.

8. Sensor und Führungsdrahteinheit gemäß Anspruch 7, wobei sich ein Bereich des Rohrsegments (24) von einem distalen Ende der Röhre aus erstreckt und die Sensor und Führungsdrahteinheit weiterhin eine Spule (15) umfasst, die mit dem Bereich des Röhrensegments, das sich von dem distalen Ende der Röhre aus erstreckt, montiert ist, wobei das Röhrensegment einen äußeren Durchmesser aufweist, der im Wesentlichen gleich zu einem inneren Durchmesser der Röhre (2) ist.

9. Sensor und Führungsdrahteinheit gemäß Anspruch 6, wobei der Kerndraht mit der Röhre in einer Presspassung montiert ist, so dass ein äußerer Durchmesser des Kerndrahtes im Wesentlichen gleich einem inneren Durchmessers der Röhre ist.

10. Sensor und Führungsdrahteinheit gemäß einem der voranstehenden Ansprüche, wobei der Kerndraht eine Längsausnehmung (28) darin aufweist, zur Aufnahme der Verdrahtung.

11. Sensor und Führungsdrahteinheit gemäß einem der Ansprüche 1 bis 10, wobei der Kerndraht eine flache Oberfläche (30) zur Aufnahme der Verdrahtung aufweist.

12. Sensor und Führungsdrahteinheit gemäß einem der Ansprüche 1 bis 10, weiterhin umfassend eine Spule (8), die mit einem Bereich des Kerndrahtes montiert ist, der sich von einem distalen Ende der Röhre aus erstreckt.

13. Sensor und Führungsdrahteinheit gemäß Anspruch 6, wobei der Kerndraht gewindemäßig mit der Röhre in Eingriff steht, wobei der Kerndraht ein äußeres Gewinde (39) umfasst, das komplementär zu einer spiralartigen Struktur, die an dem distalen Ende der Röhre eingeformt ist, ist, so dass die spiralförmige Struktur der Röhre gewindemäßig mit zumindest einigen der Gewindegänge des Kerndrahtes in Eingriff bringbar ist.

14. Sensor und Führungsdrahteinheit gemäß Anspruch 13, weiterhin umfassend eine Spule, die Spulenwicklungen aufeist, die gewindemäßig mit distalen Gewindegängen (39) des Kerndrahtes eingreifen.

## Revendications

1. Ensemble de capteur et guide souple comprenant
un guide central (1) ayant une extrémité distale, une extrémité proximale et une partie élargie (10) à l'extrémité distale, ladite partie élargie étant munie d'un élément de montage de capteur sous la forme d'une fente (11),
un capteur (12) monté dans ledit élément de montage de capteur de ladite partie élargie (10) dudit guide central (1),
**caractérisé en ce que** ledit élément de montage comprend un élément essentiellement cylindrique, ladite fente (11) étant formée dans celui-ci, et conçu pour loger ledit capteur, et **en ce que**
les parois latérales à l'intérieur de la fente (11) s'étendent sur une certaine distance au-dessus de la surface supérieure du capteur (12) de manière à fournir une protection adaptée dudit capteur vis-à-vis d'une interférence mécanique.

2. Ensemble de capteur et guide souple selon la revendication 1, dans lequel la fente (11) présente des dimensions en largeur et en profondeur dans la plage de 50 à 250 *µ*m, et une dimension en longueur dans la plage de 50 à 2 500 *µ*m.

3. Ensemble de capteur et guide souple selon la revendication 1, dans lequel ledit capteur (12) est fixé en place en incrustant ledit capteur dans un matériau mou sélectionné à partir du groupe constitué du caoutchouc de silicone, du latex, et d'autres matériaux polymères.

4. Ensemble de capteur et guide souple selon la revendication 1, dans lequel un autre creux (19) a été formé dans le fond de la partie élargie (10), en dessous de l'extrémité distale du capteur (12) afin de s'assurer que la partie distale sensible à la pression du capteur ne subira aucune contrainte mécanique.

5. Ensemble de capteur et guide souple selon la revendication 1, dans lequel ladite fente (11) dans laquelle ledit capteur est monté s'étend au travers de ladite partie élargie.

6. Ensemble de capteur et guide souple selon la revendication 1, comprenant un tube (2) entourant ledit guide central (1) sur au moins une partie de la longueur dudit guide central de sorte que ledit guide central (1) dépasse de l'extrémité distale dudit tube (2), ledit tube étant configuré pour permettre audit ensemble de capteur et guide souple d'être inséré dans une artère et d'être déplacé jusqu'à un site de mesure à l'intérieur d'un corps.

7. Ensemble de capteur et guide souple selon la revendication 6, comprenant un fil (14) s'étendant depuis ledit capteur (12) au travers d'au moins une partie dudit tube (2) en direction d'une extrémité proximale dudit tube, et
un segment de tube (24) ayant un diamètre extérieur pratiquement identique au diamètre intérieur dudit tube, ledit segment de tube étant positionné entre ledit guide central (1) et ledit tube (2) dans un ajustement serré avec le tube pour fixer ledit guide central en place dans ledit tube, et ledit segment de tube étant configuré pour permettre le passage dudit fil au travers de ladite partie dudit tube.

8. Ensemble de capteur et guide souple selon la revendication 7, dans lequel une partie dudit segment de tube (24) dépasse de ladite extrémité distale dudit tube, et ledit ensemble de capteur et guide souple comprend en outre une bobine (15) montée sur ladite partie dudit segment de tube dépassant de ladite extrémité distale dudit tube, ledit segment de tube ayant un diamètre extérieur pratiquement égal au diamètre extérieur dudit tube (2).

9. Ensemble de capteur et guide souple selon la revendication 6, dans lequel ledit guide central est monté sur ledit tube dans un ajustage serré tel qu'un diamètre extérieur dudit guide central est pratiquement égal à un diamètre interne dudit tube.

10. Ensemble de capteur et guide souple selon l'une quelconque des revendications précédentes, dans lequel ledit guide central comprend un évidement longitudinal (28) dans celui-ci destiné à loger ledit fil.

11. Ensemble de capteur et guide souple selon l'une quelconque des revendications 1 à 10, dans lequel ledit guide central comprend une surface plane (30) destinée à loger ledit fil.

12. Ensemble de capteur et guide souple selon l'une quelconque des revendications 1 à 10, comprenant en outre une bobine (8) montée sur une partie dudit guide central dépassant de l'extrémité distale dudit tube.

13. Ensemble de capteur et guide souple selon la revendication 6, dans lequel ledit guide central est engagé par filetage dans ledit tube, ledit guide central comprenant des filets externes (39) qui sont complémentaires d'une structure en spirale formée à ladite extrémité distale dudit tube de sorte que ladite structure en spirale dudit tube engage par filetage au moins quelques-uns desdits filets dudit guide central.

14. Ensemble de capteur et guide souple selon la revendication 14, comprenant en outre une bobine comportant des enroulements de bobine engageant par filetage les filets distaux desdits filets (39) dudit guide central.
